(19) **Europäisches Patentamt**

**European Patent Office**

**Office européen des brevets**

(11) Veröffentlichungsnummer: **0 238 438 B1**

(12) **EUROPÄISCHE PATENTSCHRIFT**

(45) Veröffentlichungstag der Patentschrift: **20.05.92**

(51) Int. Cl.⁵: **A61F 2/36**

(21) Anmeldenummer: **87730027.7**

(22) Anmeldetag: **13.03.87**

(54) **Knochenimplantat.**

(30) Priorität: **15.03.86 DE 3609121**
**29.07.86 DE 3625520**

(43) Veröffentlichungstag der Anmeldung:
**23.09.87 Patentblatt 87/39**

(45) Bekanntmachung des Hinweises auf die
Patenterteilung:
**20.05.92 Patentblatt 92/21**

(84) Benannte Vertragsstaaten:
**AT BE CH DE ES FR GB IT LI NL SE**

(56) Entgegenhaltungen:
**EP-A- 0 149 527**
**WO-A-85/03426**
**DE-A- 3 216 539**

(73) Patentinhaber: **MECRON MEDIZINISCHE PRO-
DUKTE GMBH
Nunsdorfer Ring 23-27
W-1000 Berlin 48(DE)**

(72) Erfinder: **Kranz, Curt, Dipl.-Ing.
Kufsteiner Strasse 12
W-1000 Berlin 62(DE)**
Erfinder: **Zahedi, Amir Dr.
Birkenweg 16
W-4400 Münster(DE)**
Erfinder: **Anapliotis, Emmanuel
Kiebitzweg 5
W-1000 Berlin 33(DE)**

(74) Vertreter: **Christiansen, Henning, Dipl.-Ing.
Patentanwalt CHRISTIANSEN Pacelliallee
43/45
W-1000 Berlin 33(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Knochenimplantat der im Oberbegriff des Anspruchs 1 genannten Art.

Derartige Knochenimplantate sind beispielsweise in Form von Hüftgelenksprothesen bekannt, welche für beidseitige Implantation geeignet sind. Um eine spezielle Anpassung an die unterschiedliche Formung der Knochenpartien der linken und rechten Körperhälfte zu erzielen, werden entweder jeweils spezielle Prothesen hergestellt, welche insgesamt von vorn herein eine der jeweiligen Körperhälfte angepaßte Form aufweisen oder es werden separat geformte Zusatzelemente nach Einfügung des spiegelsymmetrischen Grundelements nachträglich in die Knochenpartie eingebracht.

So werden in der WO-A-84/03037 die zwei sich gegenüberliegenden Seiten eines geraden blattartigen spiegelsymmetrischen Shaftes für eine Hüftgelenkprothese mit Führungen versehen, in die dann nach Einbringung des Schaftes in die Knochenpartie keilartige Klemmkörper eingeschoben werden, um eine erhöhte Sicherheit des Schaftes gegen Rotation im Knochen zu schaffen. Die Führungen werden hierbei entweder durch Aussparungen im Schaft oder an der Schaftoberfläche angebrachte herausragende Vorsprünge gebildet.

Weiterhin ist aus der EP-A-0 149 527 ein Knochenimplantat bekannt, welches am proximalen Ende eines Grundelements aufgesetzte Keilelemente aufweist, die nach Einführung des Implantats eine gute Verankerung im Knochen gewährleisten sollen.

Aus der nicht vorveröffentlichten EP-A-0 196 258 ist ein weiteres Knochenimplantat bekannt, welches auch am proximalen Ende des Grundelements aufgesetzte Zusatzelemente aufweist, die nach Einführung des Implantats eine starre Verbindung mit dem Knochen eingehen sollen. Das Grundelement und die Zusatzelemente sind derart ausgebildet, daß sich das Grundelement nach der Einbringung des Implantats in die Knochenpartie stets relativ zu den Zusatzelementen bewegen kann.

Bei den drei aufgeführten Knochenimplantaten werden die Zusatzelemente, die hauptsächlich als Keilelemente wirken, nicht einseitig zur Symmetrieebene des Grundelements sondern beidseitig der Symmetrieebene eingebracht, so daß das dadurch entstehende Knochenimplantat weiterhin im wesentlichen spiegelsymmetrisch ist. Eine Anpassung an nicht spiegelsymmetrische Knochenbereiche der linken bzw. rechten Körperhälfte findet also bei diesen bekannten Knochenimplantaten nicht bzw. nur durch die Auswahl von unterschiedlich ausgebildeten Zusatzelementen statt. Zudem gehen die Zusatzelemente dieser bekannten Knochenimplantate keine feste Verbindung mit dem Grundelement ein. Sie werden nur in den Aussparungen bzw. an der Oberfläche des Grundelements geführt und werden in Eintreibrichtung lediglich durch Einkeilung vor einer Weiterbewegung gehindert. Bei einer Lockerung des Implantats können sich die Zusatzelemente deshalb leicht verschieben, welches zu einer veränderten Lastabtragung führen kann und im äußersten Fall den Ersatz des Knochenimplantats erforderlich macht.

Der im kennzeichnenden Teil des Anspruchs 1 angegebenen Erfindung liegt daher die Aufgabe zugrunde, bei einem Knochenimplantat der eingangs genannten Gattung, welches bedingt durch das einheitlich ausgebildete Grundelement für beide Körperhälften einfach und kostengünstig hergestellt werden kann, eine Ausgestaltung der Grund- und Zusatzelemente anzugeben, welche eine bessere, sichere und dauerhaft starre Verbindung der Elemente untereinander zur Bildung einer individuell anpaßbaren nicht spiegelsymmetrischen links- oder rechtsseitigen Ausführung des Implantats ermöglicht.

Der Erfindung beruht auf der Erkenntnis, daß wenn das spiegelsymmetrische Grundelement eines Knochenimplantats im Bereich zweier einander gegenüberliegender Oberflächen eine starre Arretierung ermöglichende Befestigungselemente, insbesondere in Form von in die Außenkontur eingelassene Aussparungen oder mindestens eine durchgehende Aussparung als Bohrung, zur Verankerung des Zusatzelements aufweist, daß sich dann die Elemente auf einfache und sichere Weise vor der Einbringung des Implantats in die Knochenpartie sich zu einer individuell anpaßbaren nicht spiegelsymmetrischen links- oder rechtsseitigen Ausführung des Implantats starr miteinander sicher und dauerhaft verbinden lassen.

Durch unterschiedliche Größen der Zusatzelemente wird damit auch eine noch individuellere anatomische Anpassung möglich. Bei an einander gegenüberliegenden Seiten des Grundelements des Implantats vorgesehene Befestigungselemente für die Zusatzelemente kann aus einem Grundelement und dem entsprechenden Zusatzelement nach Belieben die nicht spiegelsymmetrische links- oder rechtsseitige Ausführung des Implantats erstellt werden.

Wenn die Befestigungselemente für zwei unterschiedliche Zusatzelemente symmetrisch zu einer das Grundelement in zwei spiegelbildliche Hälften teilenden Fläche (Symmetrieebene) angeordnet sind, können auch die Zusatzelemente für die Anwendung in der linken bzw. rechten Körperhälfte zueinander symmetrisch ausgebildet sein und sind deshalb einfach mit geringfügigen Umstellungen der Werkzeugmaschinen herstellbar.

Besonders günstig ist weiterhin die Ausbildung der Zusatzelemente in für sich ebenfalls spiegelsymmetrischer Ausführung. Die Symmetrie besteht dabei in der Draufsicht - also gesehen auf die Anschlußfläche des Implantats. Die Symmetrieebene - also der gedachte Spiegel - bildet somit eine zur Anschlußfläche des Implantats senkrecht stehende Fläche. Damit ist zur Anpassung an beide Körperhälften nur ein einziges Zusatzelement erforderlich.

Die symmetrische Form entsteht dabei im wesentlichen aus der (gedachten) Überlagerung der beiden unsymmetrischen Formen, in der Weise, daß derjenige Raumbereich, in dem sich beide Teile überschneiden, die symmetrische Form bildet. Dadurch werden Unsymmetrien im Bereich der Verbindungsfläche zum Grundelement nicht ausgeschlossen, da diese frei gestaltet werden kann - unter der Bedingung, daß die gegenüberliegende Fläche eine entsprechende Form aufweist.

Ist die Aussparung durch das Implantat hindurchgehend als Bohrung oder sonstiger (prismatischer) Durchlaß ausgebildet, läßt sie sich einfach herstellen, da dann beide Seiten des Implantats in einem einzigen Arbeitsgang bearbeitet werden können. Bei symmetrischer Gestaltung des Zusatzteils ist der Durchlaß bevorzugt ebenfalls symmetrisch zu gestalten, da dann dasselbe Profil für seine gesamte Länge benutzt werden kann.

Bei den verschiedenen Varianten wird mindestens eine Aussparung als Bohrung oder Schlitz bzw. als entlang der Oberfläche verlaufende Nut erzeugt, wobei die Eingriffsmöglichkeit für das Haltelement zur Verankerung des Befestigungselements wesentlich ist. Bei schwalbenschwanzförmigen Ausnehmungen ist das Zusatzelement einschiebbar, während es bei anderen Aussparungsformen mit zylindrischer oder sonstiger prismatischer Form einsteckbar ausgebildet ist.

Die Befestigungsrichtung, d.h. die Richtung des Einsetzens der Zusatzelemente verläuft bei Schaftprothesen oder sonstigen in den Knochen eintreibbaren Implantaten quer oder entgegengestzt zur Eintreibrichtung, d.h. insbesondere quer bzw. entgegengesetzt zur Schaftrichtung, so daß eine Lockerung auch beim kräftigen Eintreiben nicht zu befürchten ist.

Die Befestigung des Zusatzelements in der Aussparung oder Ausnehmung wird dadurch verbessert, daß sich diese in Richtung auf die Oberfläche, an der das Zusatzelement anfügbar ist, verjüngt, wobei bei durchgehend geformter Aussparung die sich verjüngenden Bereiche aneinander anschließen, so daß die resultierende Bohrung insgesamt tonnenförmig oder tailliert ist, d.h. bevorzugt ebenfalls symmetrisch zur Symmetrieebene des Grundelements ausgebildet ist. Das Zusatzelement weist entsprechend ein in Aussparung eingreifendes Halteelement auf, das sich in Richtung auf das Grundelement hin erweitert, bevorzugt auch in Hinterschneidungen eingreift bzw. elastisch einrastet. Wenn das Zusatzelement durch eine Rastverbindung mit dem Grundelement des Knochenimplantats verbunden ist, kann nach Auswahl eines geeigneten Zusatzelements das Implantat auf einfache und schnelle Weise mit dem ausgewählten Zusatzteil komplettiert werden.

Auch wenn die Aussparung rein zylindrisch oder prismatisch durchgehend ausgebildet ist, sichern spreizbare Fortsätze eine gute Haftung. Zum Spreizen der Fortsätze - und damit zur dauerhaften Befestigung des Zusatzelements - wird gleich- oder gegensinnig zur Einführungsrichtung des Zusatzelementes ein zwischen den Fortsätzen einrastbarer Keil oder Keilstumpf eingeführt, der zwischen den Fortsätzen verbleibt und diese permanent spreizt. Die Fortsätze des Zusatzelements bilden insbesondere dübelartige Befestigungen, in die zur Arretierung als Klemmelement ein Keilstift oder eine Keilschraube mit insbesondere konischem Schaft bzw. Kopf einbringbar ist.

Um eine gute Anpassung des Implantats an den Knocheninnenraum und insbesondere ein großflächiges Anliegen mit festem Sitz zu erzielen, ist es günstig, wenn das Zusatzelement in Richtung auf das Grundelement nachgiebig, insbesondere elastisch, ausgebildet ist, wobei die Nachgiebigkeit durch Aussparungen oder eine Welligkeit des das Zusatzelement bildenden, dreidimensional verformten Blechs erzeugbar ist. Dadurch, daß das Zusatzelement eine räumliche Verformung im Sinne eines wesentlichen sich vom Grundelement entfernenden Höhenabstands aufweist, kann die Nachgiebigkeit weit größer sein als bei Implantaten, die eingesetzte Blechteile enthalten, die sich parallel zur Oberfläche des Grundelements erstrecken und nicht gleichzeitig eine räumliche Anpassung bezwecken.

Wenn das Zusatzelement eine die Verbindung mit dem angrenzenden Knochenbereich durch Einwachsen begünstigende Oberflächenstruktur aufweist, die Vertiefungen aufweisende Bereiche enthält, welche mit resorbierbarem Material ausgefüllt sind, das nach dem Implantieren durch Knochenmaterial ersetzt wird, so läßt sich das Implantat wegen der glatten Oberfläche zunächst leicht einführen, während durch Ersetzen des resorbierbaren Materials durch Knochenmaterial eine innige Verbindung der Implantatoberfläche mit dem umgebenen Knochen erzielt wird.

Bei einer vorteilhaften Weiterbildung der Erfindung besteht das Zusatzelement aus dreidimensional geformten Blech, das einen Hohlraum umschließt.

Wenn der Hohlraum eine Zugangsöffnung aufweist, die an einem in Bezug auf die Einführungsrichtung beim Einbringen des Implantats rückwärtig gelegenen Teil angeordnet ist, lassen sich durch die Zugangsöffnung bei der Implantation Knochenspäne in den Hohlraum einbringen, welche ein Anwachsen begünstigen. Diese Zugangsöffnung ist bevorzugt an einem in bezug auf die Einführungsrichtung beim Einbringen des Implantats rückwärtig gelegenen Teil angeordnet, so daß sie noch nach dem Einbringen der Prothese in den Implantationsbereich noch zugänglich ist.

Um die Verbindung eines Implantats mit den angrenzenden Knochenbereichen - und insbesondere das Einwachsen - zu verbessern, ist es bekannt, die Oberfläche des Implantats mit einer Strukturierung zu versehen. Diese Struktierung wurde zunächst durch eine entsprechende Formung der Oberfläche des Grundkörpers selbst erzeugt (Aufrauhung durch nachträgliche Oberflächenbehandlung, Profilierung mit der Formgebung des Grundkörpers, Einfräsung von Schlitzen oder dergleichen). Die genannte Strukturierung wird - wie erwähnt - bei aus Metall bestehenden Implantaten verschiedentlich auch durch - gegebenenfalls zum Teil in den Grundkörper eingelassene - metallische Auf- oder Einlagen in Form von Lochblechen oder Drahtgeweben erzeugt, die mit dem Grundkörper verbunden sein können. Bei dem erfindungsgemäßen Implantat läßt sich die Oberflächenstrukturierung in günstiger Weise einfach auf dem Zusatzelement anbringen, so daß mit der Auswahl eines passenden Zusatzelements auch die Auswahl einer geeigneten Oberflächenstruktur möglich ist.

Es ist ersichtlich, daß sowohl die Nachgiebigkeit und/oder Elastizität als auch die Oberflächenstruktur - also die einen sicheren Halt im Knochen bewirkende Eigenschaften - in dem Zusatzelement vereinigt werden können, weil dieses Element einen Erhebungsbereich bildet, der von der Grundform relativ am meisten abweicht. Durch gezielte Auswahl der Form und Eigenschaften dieses Bereiches lassen sich der Sitz und die Halteeigenschaften des Implantats weitestgehend beeinflussen, so daß bei Beibehaltung des Grundelements durch lokale Anpassung über eine Auswahl eines entsprechenden Zusatzelements die erstrebten Eigenschaften des Implantats bei kleinem Material- und Lageraufwand in einem großen Bereich variiert werden können.

Zum verbesserten Eingriff in die Knochenoberfläche ist gegebenenfalls auch günstig, wenn das Zusatzelement Rippen oder Rippenteile aufweist, die in Eintreibrichtung verlaufen. Auch hier wird die Herstellung vereinfacht, wenn sich die Rippen bzw. die Rippenteile ausschließlich an dem Zusatzteil befinden, wo sie als Halte- und Führungselemente besonders wirksam sind. An dem spiegelsymmetrischen Grundelement brauchen dann keine Rippen erzeugt zu werden. Hier kann ebenfalls das Zusatzteil in verschiedenen Varianten produziert werden, so daß sich je nach Größe des mit dem Knochenimplantat zu versehenden Knochens ein passendes Zusatzteil am spiegelsymmetrischen Grundbestandteil des Knochenimplantats befestigen läßt. Bevorzugt weist das Zusatzelement Rippen oder Rippenteile auf, die in Eintreibrichtung verlaufen, und die Herstellung wird vereinfacht, wenn sich die Rippen bzw. die Rippenteile ausschließlich an dem Zusatzteil befinden, wo sie als Halte und Führungselemente besonders wirksam sind.

Dagegen kann das Zusatzteil in verschiedenen Varianten produziert werden, so daß sich je nach Größe des mit dem Knochenimplantat zu versehenden Knochens ein passendes Zusatzteil am spiegelsymmetrischen Grundbestandteil des Knochenimplantats befestigen läßt.

Die Anwendung bezieht sich bevorzugt auf Schaftprothesen für Röhrenknochen, wie Femurprothesen, wobei sich das Zusatzelement in einer Ebene erstreckt, die parallel zu derjenigen Ebene gerichtet ist, in der der Schaft seine maximale Krümmung aufweist. Durch räumliche Anpassung dieser Zone lassen sich die Sitzeigenschaften von Femurprothesen patientenindividuell anpassen und gegebenenfalls noch während der Operation variieren.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur I eine Seitenansicht eines Ausführungsbeispiels des erfindungsgemäßen Implantats in Gestalt einer Hüftgelenksprothese,

Figur 2 eine zweite Ansicht der Prothese gemäß Figur I,

Figur 3 den geschnittenen Teilbereich III des Ausführungsbeispiels gemäß Figur 2 in vergrößerter Darstellung,

Figur 4 eine Schnittdarstellung einer Variante des Ausführungsbeispiels gemäß Figur I,

Figur 5 eine vergrößerte Detaildarstellung des Bereichs V der Schnittansicht der Variante gemäß Figur 4,

Figur 6 eine Seitenansicht der Variante gemäß Figur 4,

Figuren 7 a bis c eine Ausführungsvariante mit einem symmetrischen Zusatzelement in drei Darstellungen,

Figur 8 eine perspektivische Teilansicht einer weiteren Variante des Ausführungsbeispiels gemäß Figur I,

Figur 9 eine weitere Variante des Ausführungsbeispiels gemäß Figur I in perspektivischer Darstellung sowie

Figuren 10 a bis d verschiedene Varianten von Oberflächengestaltungen insbesondere der Zusatzelemente des erfindungsgemäßen Implantats.

Das in den Figuren wiedergegebene Ausführungsbeispiel eines erfindungsgemäßen Implantats in Form einer Hüftgelenksprothese besteht (unter Bezugnahme zunächst auf Figuren I und 2) aus einem Schaft I, einem Hals 2 und einem sphärischen Gelenkkopf 3, wobei letzterer mittels eines Konusanschlusses auswechselbar ist. Die bisher genannten Teile bilden die spiegelsymmetrischen Bestandteile der Hüftgelenksprothese, wobei der Schaft dem Grundelement entspricht.

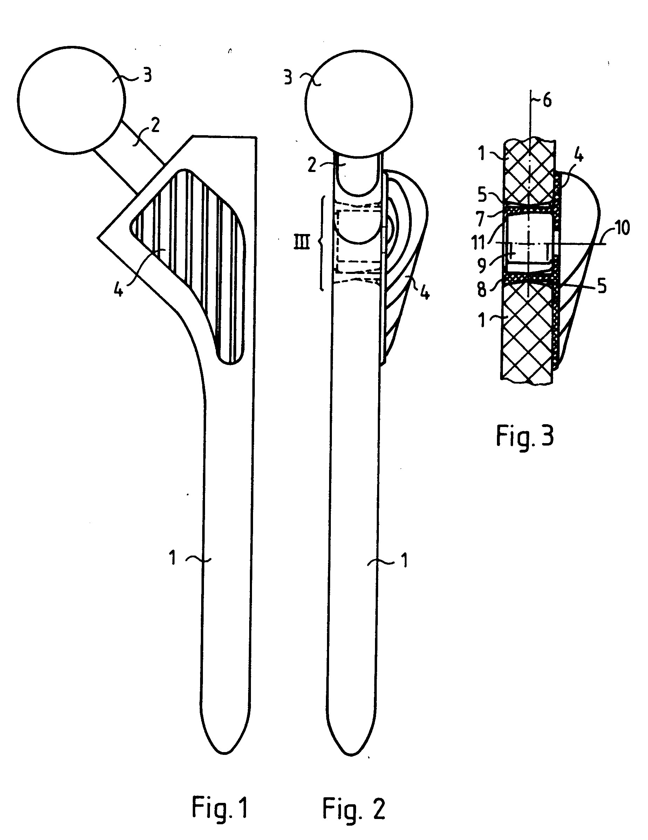

Außerdem weist die gezeigte Hüftgelenkprothese ein nachträglich anbringbares Zusatzelement 4 mit Rippen auf, die in Richtung der Eintreibrichtung, also in Richtung der Längsachse des Schaftes, verlaufen. Bei einer Hüftgelenkprothese für die andere Körperseite wird wahlweise ein spiegelsymmetrisch zu dem dargestellten geformtes Zusatzelement auf der gegenüberliegenden Seite des Schaftes gemäß Figur 2 angebracht.

Wie Figur 3 zeigt, ist in dem Schaft I als Aussparung zur Aufnahme eines Befestigungselements des Zusatzelements 4 eine Öffnung 5 in Form einer durchgehenden Bohrung vorgesehen. Die Bohrung verjüngt sich jeweils zur Mitte hin, so daß ein sich aufspreizendes Befestigungselement für ein an der gegenüberliegenden Seite vorgesehenes Zusatzelement in dem betreffenden konischen Bereich Halt findet und mittels eines dübelartigen Elements auf einfache Weise zu befestigen ist. Das Grundelement läßt sich auch ohne Zusatzelement implantieren, da keine vorspringenden Bereiche stören. Die Öffnung 5 erstreckt sich spiegelsymmetrisch zur Symmetrieebene 6. In diese Öffnung ist das Zusatzteil 4 mittels zweier angeformter Fortsätze 7 und 8 eingesetzt. Die freien Enden der Fortsätze 7 und 8 lassen sich so spreizen, daß sie die in der Symmetrieebene liegenden engste Stelle (Taille) der Öffnung 5 hintergreifen. Durch eine zentrale Bohrung als Ausnehmung bilden sie eine Art Dübel, der seine Haltekraft durch ein dort eingetriebenes Spreizelement 9 gewinnt, an das die genannte Bohrung angepaßt ist. Der Fortsatz 8 ist im aufgespreizten Zustand gezeigt, der durch einen (in der Zeichnung von links) eingetriebenen Keilstumpf 9 als Spreizelement erzwungen ist, wobei nur der unterhalb der Mittellinie 10 liegende Teil wiedergegeben ist. Die obere Hälfte ist fortgelassen, so daß der Blick in die Öffnung 5 im Schaft I im Zustand ummittelbar nach dem Einbringen des Zusatzelements 4 freigegeben ist.

Der Fortsatz 7 weist an seinem freien Ende eine Verdickung 11 auf, die verhindert, daß ein eingetriebener Keilstumpf wieder aus dem Lochbereich herausgleitet. Auf diese Weise ist eine Klemmverbindung hergestellt, mit deren Hilfe das Zusatzteil 4 dauerhaft am Schaft I befestigt werden kann. Es ist ersichtlich, daß die Fortsätze 7 und 8 derart ausgestaltet werden können, daß sie Ansätze aufweisen, die nach dem Einführen in Hinterschneidungen der Bohrungen eingreifen. In diesem Fall sind statt der konischen Bereiche entsprechende stufenförmige Zonen vorzusehen.

Es ist ersichtlich, daß ein Verklemmen oder Verkeilen des Zusatzelements auf unterschiedliche Weise möglich ist, wobei die Aussparung des Grundelements relativ glattwandig gehalten werden kann und das Halteelement (Fortsätze) zunächst nach manuellem Eindrücken einfach einrastet. Das Spreiz- oder Klemmelement bildet dann sozusagen die Verriegelung und Sicherung auch bei größerer Beanspruchung auftretenden Kräften.

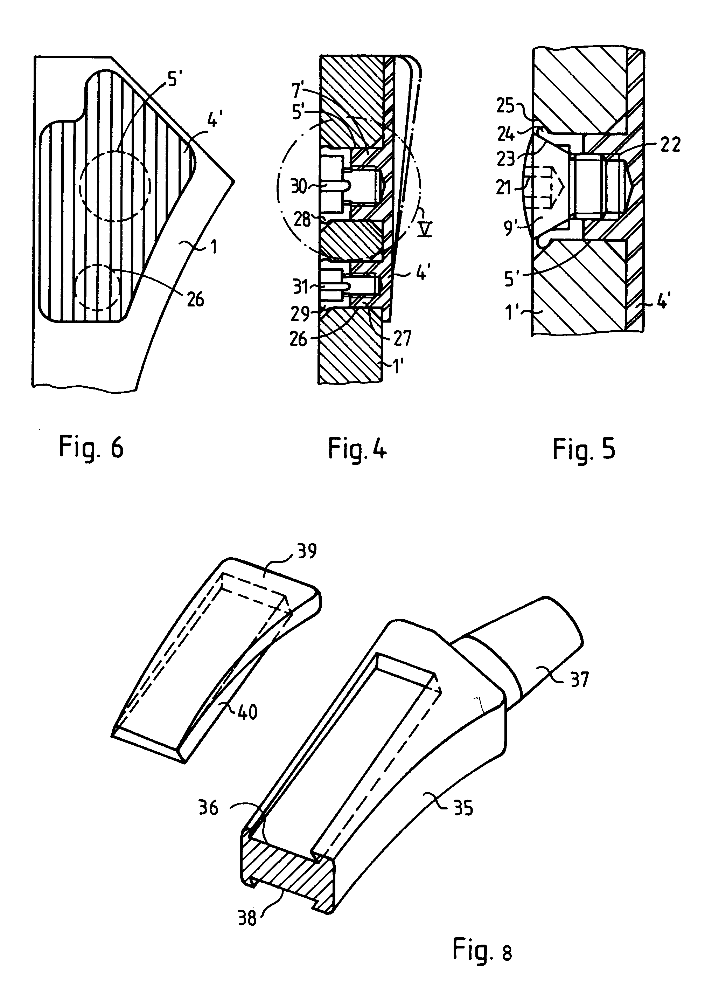

Bei dem in den Figuren 4 bis 6 in verschiedenen Ansichten wiedergegebenen Ausführungsbeispiel ist als Keilkörper eine Keilschraube 9' vorgesehen, welche einen Innensechskant 21 aufweist, mit dem die Schraube mittels eines üblichen Sechskantschlüssels ein- oder ausgedreht werden kann. Das Zusatzelement 4' weist dazu eine Bohrung mit Innengewinde 22 auf. Durch die Keilwirkung der konischen Unterseite 23 wird der obere Randbereich 24 des Zusatzteils 4' in eine konische Ausnehmung 25 der als zylindrische Bohrung 5' ausgebildeten Öffnung hineingepreßt, so daß sich der - zunächst nur geringfügig vorspringende - Rand nach Art eines Hohlniets vollends umbördelt und zusammen mit der Anpresskraft der Schraube 9' das Zusatzteil festhält.

In den Figuren 4 und 6 ist ersichtlich, daß bei dem dargestellten Ausführungsbeispiel des Schafts 1' neben der Bohrung 5' eine zusätzliche Bohrung 26 vorgesehen ist, in die ein weiterer Ansatz 27 des Zusatzteils 4' eingreift, um eine Verdrehsicherung zu bewirken.

Die Ansätze 7' und 27 sind bei diesem Ausführungsbeispiel hohlzylindrisch ausgeführt, wobei am Ende jeweils eine umlaufende Wulst 28 und 29 vorgesehen ist, welche in die konischen Erweiterungen der Bohrungen 5' bzw. 28 eingreift und die Ansätze insoweit in den Prothesenschaft einfach einschnappbar macht. Die Sicherung kann durch einen Keilkörper erfolgen, wobei dieser - wie dargestellt - hier bevorzugt als Schraube mit keilförmiger Kopfunterseite ausgebildet ist. U-förmige Ausnehmungen 30 und 31 in den zylindrischen Körpern geben diesen die notwendige Elastizität.

Die konischen Erweiterungen der Bohrungen sind symmetrisch ausgebildet, so daß ein Zusatzelement 4' in der jeweils entsprechenden Formgebung wahlweise von beiden Seiten her eingefügt werden kann. Die zweite Bohrung 26 verhindert zuverlässig ein unbeabsichtigtes Verdrehen des Zusatzelements.

Das Zusatzelement ist bevorzugt aus Titan oder einem körperverträglichen Kunststoff hergestellt. Bei der Metallausführung wird im wesentlichen kein Verquetschen des Randbereichs beim Festziehen der Keilschraube auftreten. Hier ist eine durchgehend zylindirische Ausgestaltung der Bohrungen 5' und 26 günstig. Weiterhin braucht der Ansatz 27 lediglich die Form eines Zylinders aufzuweisen, um einen Widerstand ausschließlich gegen Verdrehen darzustellen.

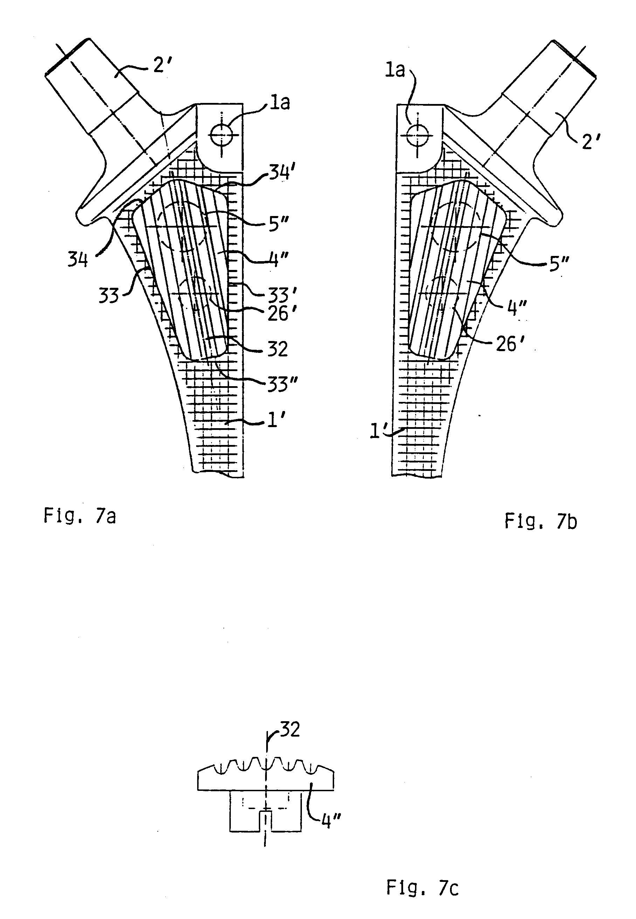

In den Figuren 7a bis 7c ist ein weiteres Ausführungsbeispiel der erfindungsgemäßen Prothese mit einem Zusatzelement 4″ dargestellt, welches symmetrisch ausgebildet ist. Die Schaftprothese 1' mit einem Konus 1″ zur Aufnahme einer Gelenkkugel weist als Aussparungen 5″ und 26' glatt durchgehende zylindrische Bohrungen auf, die mit Ausnahme einer geringfügigen Verrundung im Bereich des Übergangs zu den Außenflächen keine zusätzlichen Bearbeitungen aufweisen.

Das Zusatzelement 4″ ist bezüglich einer Achse 32 (Spiegelebene) symmetrisch ausgebildet, so daß es beidseitig verwendbar ist. Die Figuren 7a und b zeigen die Prothese in der Ansicht von beiden Seiten mit jeweils eingesetztem identischen Zusatzelement 4″. Die Bohrungen 5″ und 26' befinden sich auf der Symmetrieachse 32.

Durch Einstecken in die Ausnehmungen und Befestigen mit einer Spreizschraube (od er ähnlich, wie es anhand der zuvor dargestellten Ausführungsbeispielen beschrieben ist) läßt sich die Schaftform mit einem einzigen Zusatzelement in diejenige einer Links oder Rechts-Prothese umwandeln. Damit ist der Arzt in der Lage, ausgehend von einer einzigen Verpackungseinheit, welche den Schaft 1' und ein Zusatzteil 4″ sowie zugehörige Befestigungsmittel umfaßt, sich die gewünschte Prothesenform herzustellen.

Das Zusatzelement 4″ weist eine fünfeckige Grundform auf, wobei die beiden längsten Seitenflächen einen Winkel bilden, der im wesentlichen dem Winkel der in Richtung auf die zur Befestigung dienenden Aussparungen 5″ und 26' gesehen, seitlichen Begrenzungskanten 32 und 32' in dem diesen Ausnehmungen benachbarten Bereich entspricht. Die Symmetrieachse bildet die linke Halbierende, wobei der erweiterte Bereich durch zwei symmetrische Dachkanten 34 und 34' begrenzt ist, welche sich auf der Symmetrielinie treffen. Von den dachkantenartigen Begrenzungslinien 34 und 34' verläuft jeweils eine parallel zum Prothesenkragen oder der entsprechenden Begrenzungslinie der Prothese, d.h. senkrecht zur Achse des Befestigungskonus 2'.

Durch die dachkantartige fünfeckige Form des Zusatzelements bleibt in der Nähe der einer dachkantenartigen Begrenzungskante (34' in Figur 7a; 34 in Figur 7b) ein Bereich frei, welcher eine zusätzliche Befestigungsmöglichkeit für den Prothesenschaft, beispielsweise mittels einer Bohrung 1a bildet. Die Form des Zusatzelementes ist dabei in der Draufsicht insgesamt pentagonal, wobei den dachkantartigen Seiten 34 und 34' gegenüber eine schmale Begrenzungsseite 33″ gelegen ist, welche die Symmetrielinie 32 senkrecht schneidet.

In Figur 7c ist das Zusatzelement 4″ im Schnitt wiedergegeben. Die Schnittrichtung verläuft dabei senkrecht zur Symmetrieebene 32. Das Element 4″ ist konvex gewölbt und mit Rillen versehen, wobei auch die Form der Wölbung und Rillengestaltung symmetrisch zur Ebene 32. Im Bereich des Schnittes mit dieser Ebene 32 hat die Wölbung auch ihren Scheitel, d.h. ihren am höchsten gelegenen Bereich.

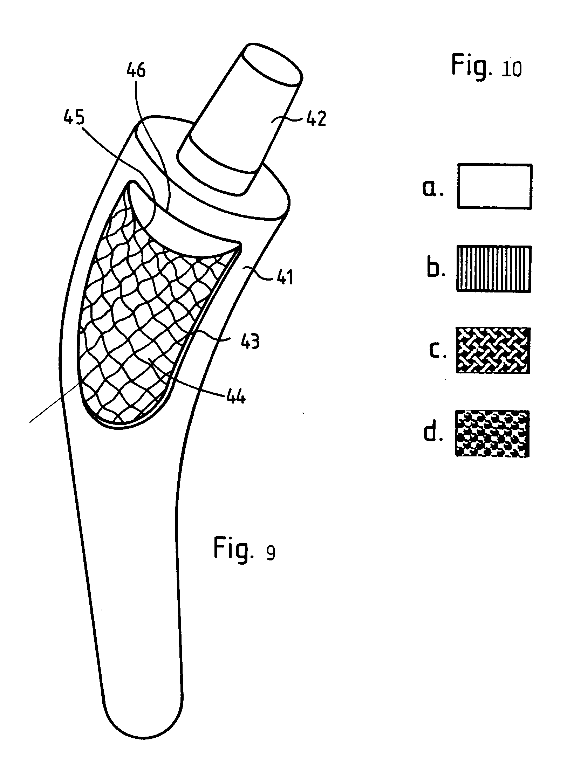

In Figur 8 ist ein weiteres Ausführungsbeispiel des erfindungsgemäßen Implantats in Form einer Schaftprothese dargestellt.

In dem Schaft 35 als Grundelement ist eine schwalbenschwanzförmige Aussparung 36 vorgesehen, welche in Richtung zum Schaftende, das dem Konus 37 gegenüberliegt, an Tiefe abnimmt und mit seiner Grundfläche die Außenfläche der Kontur des Schafts schneidet. Die Ausnehmung weist also einen schwalbenschwanzförmigen Querschnitt auf, der vom Schaftende her auf einer geraden Bahn in die Oberfläche des Schaftes eintritt und mit zunehmender Annäherung an das den Hals 37 tragende Ende des Schafts tiefer eindringt. Die Grundfläche der schwalbenschwanzförmigen Ausnehmung 36 und einer dazu symmetrisch auf der gegenüberliegenden Seite des Schaftes angeordneten Ausnehmung 38 erstrecken sich somit parallel zu derjenigen Ebene Symmetrieebene) des Schaftes, in der seine größte Krümmung gelegen ist.

Ein Ansatzteil 39 als Zusatzelement weist einen (positiv geformten) schwalbenschwanzförmigen Ansatz 40 auf, der in die Aussparung 36 einschiebbar ist. Der schwalbenschwanzförmige Ansatz 40 weist entsprechend der Ausnehmung 36 eine sich entgegengesetzt zur Einführungsrichtung verringernde Höhe auf, so daß das Element 39 in eingeschobenem Zustand an der an die Aussparung 36 sich beidseitig anschließenden Oberfläche des Schaftes 35 bündig anliegt.

Ein symmetrisch zu dem Teil 39 geformtes Element läßt sich alternativ entsprechend in die Aussparung 38 einfügen. Auf diese Weise kann vor dem Einbringen des Schaftes durch Einfügen des Elementes 39 oder eines symmetrisch geformten eine Links-oder Rechtsprothese hergestellt werden. Die beim Eintreiben des Prothesenschaftes auftretenden Kräfte halten das Element 39 in seiner Position. Gegen ein unbeabsichtigtes Lösen kann zusätzlich ein (nicht dargestellter) Verriegelungsstift in Querrichtung vorgesehen werden, welcher nach dem Einschieben des Elementes eingefügt wird und beispielsweise die Form eines Kerbstiftes hat.

Gegen ein unbeabsichtigtes Lösen beim Eintreiben des Prothesenschaftes ist die Anordnung dadurch gesichert, daß das Zusatzelement entgegen der Eintreibrichtung des Schaftes - also in Richtung seiner konischen oder sonstigen Erweiterung eingetrieben wird. Dadurch, daß die Ausnehmung sackförmig endet, ist das Zusatzelement durch den so gebildeten Anschlag gegen ein unbeabsichtigtes Lösen während des Eintreibens gesichert.

Die in Figur 9 dargestellte Hüftgelenkprothese mit einem Grundelement 41 weist einen konusförmigen Zapfen 42 zur Aufnahme einer Gelenkkugel und beidseitig in einer Ebene im wesentlichen parallel zur Symmetrieebene gerichtete vertiefte Bereiche (Ausnehmung) 43 auf, die um ca. 0,5 bis l mm abgesenkt sind (entsprechend der Stärke des Materials der später zu beschreibenden Einlage). Der Schaft der Prothese weist einen ovalen bis ellipsoiden oder ovoiden Querschnitt auf, wobei die Gestaltung im Hinblick auf die Ebene, in der die stärkste Krümmung des Schaftes verläuft, symmetrisch ist. Der Schaft kann daher in seiner Grundform als Grundelement sowohl für rechts- als auch für linksseitige Anwendung herangezogen werden. Der vertiefte Bereich 43 ist bis zu seiner Kante hin eben, wobei die Kante selbst im wesentlichen senkrecht zur Oberfläche der Prothese gerichtet ist, so daß die Einlage 44, welche in den vertieften Bereich 43 eingesetzt und mit der Randzone verschweißt ist, ohne wesentliche Stufe bezüglich der äußeren Oberfläche an die übrigen Oberflächenteile des Schaftes angrenzt - soweit es die beiden Seitenkanten und den unteren dem Konus 42 gegenüberliegenden Bereich betrifft.

Der dem Konus zugewandte Bereich der Einlage 44 (in der Zeichnung die Kante 45), schließt nicht an die entsprechende Kante 46 der Ausnehmung 43 an. Hier verbleibt eine Öffnung, welche es dem Chirurgen gestattet, Kochenspäne in den entstehenden Hohlraum einzufüllen, der einerseits eine Verdichtung des Prothesenbereichs sorgt und andererseits das Einwachsen begünstigt.

Sowohl der Schaft der Prothese 41 als auch die Einlage 44 bestehen aus Titan. Die Einlage 44 wird aus einem zunächst im wesentlichen ebenen Blech erzeugt, das aufgrund durchgehender Öffnungen oder Aussparungen bzw. einer wellenförmigen Grundstruktur durch Tiefziehen leicht dreidimensional verformbar ist. Diese Struktur erhöht einerseits die Verformbarkeit und verbessert andererseits auch den Verbund mit dem Knochen, wobei in den Hohlraum eingefügte Knochenspände durch Materialdurchbrüche hindurch mit dem umgebenden Knochen verwachsen.

Die sich somit ergebende aufgewölbte Struktur der Einlage 44 paßt sich somit den anatomischen Gegebenheiten an und sorgt für einen sicheren Sitz des Schaftes. Durch die Auffüllung mit Knochenspänen verfestigt sich im Zuge des Einwachsens die Struktur, so daß die Prothese auch bei stoßartigen Belastungen ihren sicheren Sitz behält.

Der Rand der Einlage 44 ist im Bereich ihrer Kante (mit Ausnahme des Kantenbereichs 45) mit dem angrenzenden Schaft verschweißt, so daß die Struktur insgesamt eine große homogene Festigkeit erreicht.

Die Einlage 44 kann auf verschiedene Weise so gestaltet sein, daß sie einerseits beim Einführen des Schaftes verformbar ist und so für eine großflächige Anlage am Knochen sorgt und andererseits durch die Art der Oberflächengestaltung eine innige Verbindung mit diesem Knochenmaterial eingangen wird. Die Wölbung sorgt weiterhin für eine gute räumliche Anpassung der Form und damit für guten Sitz.

Damit ergibt sich eine gute Kräfteverteilung und eine gleichmäßige Flächenbelastung des umgebenden Knochenraums. Die Einlage 45 ist ebenfalls derart dreidimensional verformt, daß sich im mit dem Schaft verschweißten Zustand im halsnahen Bereich eine Öffnung ergibt, in die Knochenspäne eingefüllt werden kann.

Die Einlage 44 ist in verschiedenen Ausführungen verfügbar, so daß - ausgehend von einem einzigen symmetrischen Schaft - durch Zufügen der jeweiligen Einlage die entsprechende Links- bzw. Rechtsausführung der Prothese ohne zusätzliche Schmiedeform erzeugt werden kann. Auch lassen sich Varianten durch unterschiedliche Auswölbung der Einlagebereiche ohne weiteres unmittelbar vor der Implantation erstellen, so daß auch insoweit eine Reduzierung der notwendigen Herstellungswerkzeuge und des Lagerraumes möglich ist.

In Figur 10 sind verschiedene Oberflächen dargestellt, welche zusammen mit den in den vorangehenden Figuren dargestellten Zusatzelementen verwendet werden können. Dabei zeigt die Figur 10a eine glatte Oberfläche, Figur 10b eine in Längsrichtung des Schaftes gerippte oder gewellte Form. Figur 10c zeigt ein Flechtmuster und Figur 10d eine mikroporöse Struktur mit feinporigen Öffnungen. Weitere bevorzugte Oberflächenstrukturen sind die Madreporierung durch Anlagerung von kleinen kugelartigen Elementen an der Oberfläche oder andere Strukturen, welche die Haftung der Prothese verbessern und das Einwachsen im Knochen begünstigen. Letzteres trifft insbesondere zu, wenn Aussparungen oder Vertiefungen der Oberfläche des Zusatzelements mit resorbierbarem Material gefüllt sind, welches im Körper durch Knochensubstanz ersetzt wird. Apatitkeramik ist ein derartiges Material, welches für Dauerimplantationen geeignet ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Knochenimplantat, insbesondere Schaftprothese, das ein spiegelsymmetrisches Grundelement aufweist und beidseitig symmetrisch mit Befestigungselementen als Anschluß für separat geformte Zusatzelemente versehen ist, **dadurch gekennzeichnet,**
daß zur Anpassung an - für sich genommen - nicht spiegelsymmetrische Knochenbereiche der linken bzw. rechten Körperhälfte nur ein Zusatzelement (4, 4', 44) an das Grundelement angefügt ist, wodurch ein nicht spiegelsymmetrisches links- bzw. rechtsseitig im Körper verwendbares Knochenimplantat entsteht, und daß das Zusatzelement durch einen Anschlag gegen ein unbeabsichtigtes Lösen entgegen der Eintreibrichtung am Grundelement gesichert ist.

2. Knochenimplantat nach Anspruch 1, **dadurch gekennzeichnet,** daß die Befestigungselemente als Aussparungen oder als Durchbrüche ausgestaltet sind.

3. Knochenimplantat nach Anspruch 2, **dadurch gekennzeichnet,** daß die Befestigungselemente (5, 5', 26, 36, 38) in die Außenkontur eingelassen sind, wobei jeweils ein Befestigungselement durch mindestens eine Aussparung gebildet wird.

4. Knochenimplantat nach Anspruch 3, **dadurch gekennzeichnet,** daß die Aussparung als Bohrung oder Schlitz bzw. entlang der Oberfläche verlaufende, und insbesondere nach unten geöffnete, Nut ausgebildet ist.

5. Knochenimplantat nach Anspruch 3, **dadurch gekennzeichnet,** daß die Aussparung sich in Richtung auf die Oberfläche, an der das Zusatzelement anfügbar ist, verjüngt und das Zusatzelement ein in die Aussparung eingreifendes Halteelement (40, 7, 8, 7', 27) aufweist, das sich in Richtung auf das Grundelement hin erweitert.

6. Knochenimplantat nach Anspruch 5, **dadurch gekennzeichnet,** daß das Halteelement elastische, insbesondere spreizbare oder sich selbsttätig aufgrund ihrer Eigenspannung spreizende Fortsätze (7, 8) aufweist.

7. Knochenimplantat nach einem der vorangehenden Ansprüche, **dadurch gekennzeichnet,** daß die Halteelemente vorspringende Rastmittel (28) aufweisen, die in entsprechende Hinterschneidungen (25) der Aussparungen des Grundelements eingreifen.

8. Knochenimplantat nach einem der vorangehenden Ansprüche, **dadurch gekennzeichnet,** daß das Halteelement als Spreizdübel ausgebildet ist, mit einer Ausnehmung zum Einbringen eines Spreizelements (9, 9') zum Aufspreizen der Fortsätze, das insbesondere als Keilstift (9) oder als Keilschraube (9') mit konischem Schaft bzw. Kopf ausgebildet ist.

9. Knochenimplantat nach einem der vorangehenden Ansprüche, **dadurch gekennzeichnet,** daß zur Verdrehsicherung des Zusatzelements eine weitere Aussparung (26) des Grundelements mit einem in diese eingreifenden weiteren Halteelement des Zusatzelements exzentrisch in bezug auf die erste Aussparung (5') vorgesehen ist.

10. Knochenimplantat nach einem der vorangehenden Ansprüche, **dadurch gekennzeichnet,** daß das Zusatzelement (44) - vorzugsweise in Richtung auf das Grundelement (41) - nachgiebig, insbesondere elastisch, ausgebildet ist.

11. Knochenimplantat nach einem der vorangehenden Ansprüche, **dadurch gekennzeichnet,** daß das Zusatzelement ein Blechteil umfaßt, welches einen Hohlraum umschließt.

12. Knochenimplantat nach Anspruch 11, **dadurch gekennzeichnet,** daß der Hohlraum eine Zugangsöffnung (45) aufweist, die an einem in Bezug auf die Einführungsrichtung beim Einbringen des Implantats rückwärtig gelegenen Teil angeordnet ist.

13. Knochenimplantat nach Anspruch 12, **dadurch gekennzeichnet,** daß die Zugangsöffnung dadurch gebildet ist, daß das Blech im Bereich der Zugangs-Öffnung in einem Abstand vom Grundelement endet.

14. Knochenimplantat nach Anspruch 11, **dadurch gekennzeichnet,** daß das Blech (44) in seinem Randbereich mit dem Grundelement (41) verschweißt oder in sonstiger Weise metallurgisch verbunden ist.

15. Knochenimplantat nach Anspruch 10, **dadurch gekennzeichnet,** daß die Nachgiebigkeit durch Materialschwächungen oder eine Welligkeit des das Zusatzelement bildenden Blechs erzeugt wird.

16. Knochenimplantat nach einem der vorangehenden Ansprüche, **dadurch gekennzeichnet,** daß das Zusatzelement eine die Verbindung mit dem angrenzenden Knochenbereich begünstigende rippenförmige, madréporierte, poröse oder sonstige Vertiefungen aufweisende Bereiche enthaltende Oberflächenstruktur aufweist, wobei insbesondere die Vertiefungen mit resorbierbarem Material ausgefüllt sind, welches nach dem Implantieren durch Knochenmaterial ersetzt wird.

17. System für ein Knochenimplantat nach einem der vorangehenden Ansprüche, **dadurch gekennzeichnet,** daß mehrere Zusatzelemente mit einheitlich geformten Befestigungselementen vorgesehen sind, welche sich bezüglich Größe, Nachgiebigkeit und/oder Oberflächenstruktur unterscheiden.

18. Knochenimplantat nach einem der vorangehenden Ansprüche zur Anwendung für eine Schaftprothese, **dadurch gekennzeichnet,** daß die Anschlußfläche für das Zusatzelement sich in einer Ebene erstreckt, die parallel zu derjenigen Ebene gerichtet ist, in der der Schaft seine maximale Krümmung aufweist.

19. Knochenimplantat nach einem der vorangehenden Ansprüche zur Anwendung für eine Schaftprothese, **dadurch gekennzeichnet,** daß die Anschlußfläche für das Zusatzelement sich in einem Bereich erstreckt, in der der Schaft seine maximale Krümmung aufweist.

20. Knochenimplantat nach einem der vorangehenden Ansprüche, **dadurch gekennzeichnet,** daß das Zusatzelement mindestens außerhalb der Anschlußfläche spiegelsymmetrisch ausgebildet ist, wobei die Symmetrieebene senkrecht zur Anschlußfläche gerichtet ist.

21. Knochenimplantat nach Anspruch 20, **dadurch gekennzeichnet,** daß das Zusatzelement fünfeckig ausgebildet ist.

22. Knochenimplantat nach Anspruch 21, **dadurch gekennzeichnet,** daß die Symmetrieachse im Scheitel einer zylindrischen Wölbung verläuft.

23. Knochenimplantat nach Anspruch 21, **dadurch gekennzeichnet,** daß Längsrippen der Außenoberfläche parallel zur Symmetrieebene verlaufen.

## Claims

1. Bone implant, particularly a stem prosthesis, which has a main member having mirror symmetry and is symmetrically provided on both sides with fastening means for the connection of separately formed added members, characterised in that, for the purpose of adaptation to bone regions of the left-hand or right-hand half of the body which considered by themselves do not possess mirror symmetry, only one added member (4, 4', 44) is attached to the main member, whereby a bone implant not possessing mirror symmetry and utilisable on the left-hand or right-hand side of the body is formed, and in that the added member is secured by means of a stop to the main member against accidental detachment counter to the direction in which it is driven in.

2. Bone implant according to Claim 1, characterised in that the fastening means are in the form of recesses or apertures.

3. Bone implant according to Claim 2, characterised in that the fastening means (5, 5', 26, 36, 38) are let into the outer contour, one fastening means in each case being formed by at least one recess.

4. Bone implant according to Claim 3, characterised that the recess is in the form of a bore or slot or of a channel extending along the surface and in particular open at the bottom.

5. Bone implant according to Claim 3, characterised in that the recess narrows in the direction of the surface to which the added member is attachable, and the added member has a retaining member (40, 7, 8, 7', 27) widening towards the main member and engaging in the recess.

6. Bone implant according to Claim 5, characterised in that the retaining member has extensions (7, 8) which are elastic, in particular capable of being spread out or which spread out automatically because of their internal stress.

9

7. Bone implant according to one of the preceding claims, characterised in that the retaining members have projecting catch means (28) which engage in corresponding undercuts (25) in the recesses in the main member.

8. Bone implant according to one of the preceding claims, characterised in that the retaining member is in the form of a straddling dowel, with a recess for the insertion of a spreader member (9, 9') for spreading out the extensions, said spreader member being in particular in the form of a wedge pin (9) or wedge screw (9') having a conical stem or head.

9. Bone implant according to one of the preceding claims, characterised in that, in order to secure the added member against turning, an additional recess (26) is provided in the main member, eccentric in relation to the first recess (5'), with a further retaining member of the added member engaging in it.

10. Bone implant according to one of the preceding claims, characterised in that the added member (44) is made resilient, particularly elastic, preferably in the direction of the main member (41).

11. Bone implant according to one of the preceding claims, characterised in that the added member comprises a sheet metal part surrounding a hollow space.

12. Bone implant according to Claim 11, characterised in that the hollow space has an access opening (45) which is disposed on a part situated at the rear in relation to the direction of introduction in the insertion of the implant.

13. Bone implant according to Claim 12, characterised in that the access opening is formed through the fact that in the region of said opening the sheet metal ends at a distance from the main member.

14. Bone implant according to Claim 11, characterised in that in its edge region the sheet metal (44) is welded or otherwise metallurgically joined to the main member (41).

15. Bone implant according to Claim 10, characterised in that the resiliency is produced by weakenings of the material or corrugation of the sheet metal which forms the added member.

16. Bone implant according to one of the preceding claims, characterised in that the added member has a surface structure which assists connection to the adjoining bone region and which contains rib-shaped, madreporiform, or porous regions or regions provided with other depressions, the depressions being in particular filled with resorbable material which is replaced by bone material after implantation.

17. System for a bone implant according to one of the preceding claims, characterised in that a plurality of added members having uniformly shaped fastening means are provided which differ in respect of size, resiliency and/or surface structure.

18. Bone implant according to one of the preceding claims for use for a stem prosthesis, characterised in that the connection surface for the added member extends in a plane directed parallel to the plane in which the stem has its maximum curvature.

19. Bone implant according to one of the preceding claims for use for a stem prosthesis, characterised in that the connection surface for the added member extends in a region in which the stem has its maximum curvature.

20. Bone implant according to one of the preceding claims, characterised in that the added member is shaped with mirror symmetry at least outside the connection surface, the plane of symmetry being directed at right angles to the connection surface.

21. Bone implant according to Claim 20, characterised in that the added member is in the form of a pentagon.

22. Bone implant according to Claim 21, characterised in that the axis of symmetry extends in the apex of a cylindrical curvature.

23. Bone implant according to Claim 21, characterised in that longitudinal ribs on the outer surface extend parallel to the plane of symmetry.

**Revendications**

1. Implant osseux, en particulier prothèse en forme de tige, qui présente un élément de base à symétrie spéculaire et qui est muni symétriquement des deux côtés d'éléments de fixation pour assurer le raccord avec des éléments supplémentaires formés séparément, caractérisé en ce que pour l'adaptation à des zones osseuses de la moitié gauche ou droite du corps n'ayant pas par elles-même une symétrie spéculaire, un seul élément additionnel (4, 4', 44) est associé à l'élément de base, ce qui fait apparaître un implant osseux sans symétrie spéculaire utilisable du côté gauche ou du côté droit dans le corps, et en ce que l'élément additionnel est assuré contre un desserrage non intentionnel en sens contraire de la direction d'enfoncement, au moyen d'une butée sur l'élément de base.

2. Implant osseux selon la revendication 1, caractérisé en ce que les éléments de fixation sont en forme d'évidements ou de percements.

3. Implant osseux selon la revendication 2, caractérisé en ce que les éléments de fixation (5, 5', 26, 36, 38) sont encastrés dans le contour externe, un élément de fixation étant à chaque fois formé par au moins un évidement.

4. Implant osseux selon la revendication 3, caractérisé en ce que l'évidement est en forme de percement ou de fente, ou de rainure courant le long de la surface, et en particulier ouverte vers le bas.

5. Implant osseux selon la revendication 3, caractérisé en ce que l'évidement se rétrécit en direction de la surface à laquelle l'élément additionnel peut être adjoint, et en ce que l'élément additionnel présente un élément de retenue (40, 7, 8, 7', 27) venant en prise dans l'évidement, et qui s'élargit en direction de l'élément de base.

6. Implant osseux selon la revendication 5, caractérisé en ce que l'élément de retenue présente des appendices (7, 8) élastiques, en particulier pouvant être écartés ou s'écartant automatiquement de par leur tension propre.

7. Implant osseux selon l'une des revendications précédentes, caractérisé en ce que les éléments de retenue présentent des organes d'arrêt saillants (28) qui vient en prise dans des découpes arrière correspondantes (25) des évidements.

8. Implant osseux selon l'une des revendications précédentes, caractérisé en ce que l'élément de retenue est conformé en cheville d'écartement, avec un évidement pour l'introduction d'un élément d'écartement (9, 9') pour écarter les appendices, qui est réalisé en particulier sous la forme d'une goupille de calage (9) ou d'une vis de calage (9') avec tige ou tête conique.

9. Implant osseux selon l'une des revendications précédentes, caractérisé en ce que pour assurer l'élément additionnel contre la torsion, il est prévu un autre évidement (26) de l'élément de base avec un autre élément de retenue de l'élément additionnel venant en prise dans cet élément de base et disposé de façon excentrique par rapport au premier évidement (5').

10. Implant osseux selon l'une des revendications précédentes, caractérisé en ce que l'élément additionnel (44) est formé de manière flexible, en particulier élastique, de préférence dans la direction de l'élément de base (41).

11. Implant osseux selon l'une des revendications précédentes, caractérisé en ce que l'élément additionnel comprend une partie métallique qui entoure un espace creux.

12. Implant osseux selon la revendication 11, caractérisé en ce que l'espace creux présente une ouverture de passage (45) qui est disposée sur une pièce inversée par rapport à la direction d'introduction lors de la pose de l'implant.

13. Implant osseux selon la revendication 12, caractérisé en ce que l'ouverture de passage est formée de manière que le métal dans la zone de l'ouverture de passage se termine à un certain intervalle de l'élément de base.

14. Implant osseux selon la revendication 11, caractérisé en ce que le métal ou tôle (44) est soudé dans sa partie marginale avec l'élément de base (41) ou y est relié d'une autre manière par un moyen métallurgique.

15. Implant osseux selon la revendication 10, caractérisé en ce que la flexibilité est produite par des affaiblissements de la matière ou une ondulation de la tôle formant l'élément additionnel.

16. Implant osseux selon l'une des revendications précédentes, caractérisé en ce que l'élément additionnel présente une structure superficielle contenant des zones facilitantes présentant des évidements en forme de rebords, en madrépores, poreux ou autres au voisinage de la zone osseuse, où en particulier les évidements sont remplis d'une matière résorbable, qui après l'implantation est remplacée par une matière osseuse.

17. Système pour implant osseux selon l'une des revendications précédentes, caractérisé en ce que sont prévus plusieurs éléments additionnels avec éléments de fixation de même forme, qui se différencient par leur taille, leur flexibilité et/ou leur structure superficielle.

18. Implant osseux selon l'une des revendications précédentes pour application dans une prothèse en forme de tige, caractérisé en ce que la surface de raccord pour l'élément additionnel s'étend dans un plan qui est parallèle au plan dans lequel la tige présente sa courbure maximale.

19. Implant osseux selon l'une des revendications précédentes pour application à une prothèse en forme de tige, caractérisé en ce que la surface de raccord pour l'élément additionnel s'étend dans une zone dans laquelle la tige présente sa courbure maximale.

20. Implant osseux selon l'une des revendications précédentes, caractérisé en ce que l'élément additionnel est formé avec une symétrie spéculaire au moins à l'extérieur de la surface de raccord, le plan de symétrie étant dirigé perpendiculairement à la surface de raccord.

21. Implant osseux selon la revendication 20, caractérisé en ce que l'élément additionnel a une forme pentagonale.

22. Implant osseux selon la revendication 21, caractérisé en ce que l'axe de symétrie est tracé dans le sommet d'une courbure cylindrique.

23. Implant osseux selon la revendication 21, caractérisé en ce que les rainures longitudinales de la surface externe courent parallèlement au plan de symétrie.

Fig. 1          Fig. 2

Fig. 3

Fig. 6

Fig. 4

Fig. 5

Fig. 8

Fig. 7a

Fig. 7b

Fig. 7c

15

Fig. 10

Fig. 9

a.

b.

c.

d.